# FASCICULE DE BREVET EUROPEEN

(11) **EP 2 376 621 B1**
(45) Date de publication et mention de la délivrance du brevet: **15.08.2018**
(21) Numéro de dépôt: 09806101.3
(22) Date de dépôt: 22.12.2009
(51) Int. Cl.: C12N 1/20, C12N 9/24, C07H 11/00, C08B 37/00, C12R 1/20

(54) **SOUCHE BACTERIENNE ET MELANGE BACTERIEN A ACTIVITE FUCANOLYTIQUE**
BAKTERIENSTAMM SOWIE MISCHUNG AUS BAKTERIEN MIT FUCAN ABBAUENDER AKTIVITÄT
BACTERIAL STRAIN AND BACTERIAL MIXTURE WITH A FUCANOLYTIC ACTIVITY

(30) Priorité: 23.12.2008 FR 0859041
(43) Date de publication de la demande: 19.10.2011
(73) Titulaire: Centre Etude Valorisation Algues, 22610 Pleubian (FR); Centre National de la Recherche Scientifique, 75016 Paris (FR); Universite d'Evry Val d'Essonne, 91000 Evry (FR)
(72) Inventeur: DANIEL, Régis, F-92130 Issy Les Moulineaux (FR); GORNARD, Sophie, F-38530 Pontcharra (FR); HEMON, Erven, F-22500 Paimpol (FR); LOGNONE, Vincent, F-35400 Saint Malo (FR); MARFAING, Hélène, F-22740 Lezardieux (FR)
(74) Mandataire: Tezier Herman, Béatrice
(86) Numéro de dépôt international: PCT/FR2009/052671
(87) Numéro de publication internationale: WO 2010/072977

(56) Documents cités:
- EP-A1- 0 870 771
- SAKAI TAKESHI ET AL: "Structures of oligosaccharides derived from Cladosiphon okamuranus fucoidan by digestion with marine bacterial enzymes." MARINE BIOTECHNOLOGY (NEW YORK), vol. 5, no. 6, novembre 2003 (2003-11), pages 536-544, XP002577334 ISSN: 1436-2228
- SAKAI T ET AL: "A MARINE STRAIN OF FLAVOBACTERIACEAE UTILIZES BROWN SEAWEED FUCOIDAN" MARINE BIOTECHNOLOGY, SPRINGER VERLAG, NEW YORK, NY, US, vol. 4, no. 4, 1 juillet 2002 (2002-07-01) , pages 399-405, XP001159693 ISSN: 1436-2228
- DANIEL R ET AL: "Degradation of algal (Ascophyllum nodosum) fucoidan by an enzymatic activity contained in digestive glands of the marine mollusc Pecten maximus" CARBOHYDRATE RESEARCH, PERGAMON, GB LNKD- DOI:10.1016/S0008-6215(99)00223-2, vol. 322, no. 3-4, 12 décembre 1999 (1999-12-12), pages 291-297, XP004184153 ISSN: 0008-6215
- DANIEL ET AL: "Electrospray ionization mass spectrometry of oligosaccharides derived from fucoidan of Ascophyllum nodosum", CARBOHYDRATE RESEARCH, PERGAMON, GB, vol. 342, no. 6, 27 February 2007 (2007-02-27), pages 826-834, XP005907760, ISSN: 0008-6215, DOI: 10.1016/J.CARRES.2007.01.009
- VALÉRIE DESCAMPS ET AL: "Isolation and Culture of a Marine Bacterium Degrading the Sulfated Fucans from Marine Brown Algae", MARINE BIOTECHNOLOGY, SPRINGER-VERLAG, NE, vol. 8, no. 1, 1 January 2006 (2006-01-01) , pages 27-39, XP019368184, ISSN: 1436-2236

### SEQUENCE LISTING

<110> Centre Etude Valorisation Algues - CNRS - Université d'Evry
<120> Souche bactérienne et mélange bactérien à activité fucanolytique
<130> B817PC00
<160> 2
<170> PatentIn version 3.3
<210> 1
   <211> 382
   <212> DNA
   <213> Flavobacterium sp.
<400> 1
   tgggaacagc gttatccgga tcttgggttt aagggtccgt aggtggatga ttaagtcaga     60
   ggtgaaatct tgcagctcaa ctgtaaaatt gcctttgata ctggttatct tgagttatta    120
   tgaagtagtt agaatatgta gtgtagcggt gaaatgcata gatattacat agaataccaa    180
   ttgcgaaggc agattactaa taatcaactg acactgatgg acgaaagcgt gggtagcgaa    240
   caggattaga taccctggta gtccacgccg taaacgatgg ttactagctg ttcggacttc    300
   ggtctgagtg gctaagcgaa agtgataagt aacccacctg gggagtacgt tcgcaagaat    360
   gaaactcaaa ggaattgacg ga                                             382
<210> 2
   <211> 227
   <212> DNA
   <213> Flavobacterium sp.
<400> 2
   gtcgtctcta cttttcggga tagcccagag aatttggatt aatgcctcat agtatataga     60
   aatggcatca tttttatatt aaagatttat cggtaaaaga tgagcatgcg ttctattagt    120
   tagttggtaa ggtaacggct taccaagacc gcgatagata ggggtcctga gagggagatc    180
   ccccacactg gtactgagac acggaccaga ctcctacggg aggcaga                  227

## Description

La présente invention concerne une souche bactérienne productrice d'enzymes à activité fucanolytique, un mélange bactérien comprenant ladite souche, l'utilisation de ladite souche ou d'un mélange bactérien la comprenant pour la production d'enzymes à activité fucanolytique, les enzymes obtenues par la culture de ladite souche, un procédé de dépolymérisation du fucane en mettant en oeuvre ladite souche ou un mélange bactérien la comprenant ou les enzymes obtenues et les fragments de fucane obtenus par ce procédé.

### DESCRIPTION DE L'ART ANTERIEUR

Le fucane est un polysaccharide de haut poids moléculaire à base de L-fucose constitutif de la paroi des algues brunes. Les polysaccharides issus des algues ont de nombreuses applications, notamment à titre d'épaississants, gélifiants, stabilisants, filmogènes, échangeurs d'ions, ou hydratants. Diverses activités biologiques du fucane et de ses fractions de plus faible poids moléculaire ont été étudiées. Le fucane est ainsi connu pour ses activités biologiques analogues à celles de polysaccharides sulfatés d'origine animale comme l'héparine et la chondroïtine sulfate. Cette propriété ouvre la voie à des applications à haute valeur ajoutée notamment dans le domaine thérapeutique (anticoagulant; antithrombotique; cytostatique; antiviral; blocage des réponses immunitaires, maladie d'Alzheimer). En Asie, un certain nombre d'études a montré l'intérêt de les intégrer au régime alimentaire.

Afin de bénéficier des propriétés intrinsèques du fucane de manière industrielle, les recherches actuelles portent sur la production de molécules dérivant du fucane et présentant des caractéristiques structurales définies et le plus reproductible possible. Ces études cherchent également à maintenir, voire augmenter, l'efficacité du composé naturel de départ. En outre, le poids moléculaire élevé du fucane peut être rédhibitoire pour le développement d'un actif pharmaceutique, notamment à cause d'un caractère immunogène potentiel ou de sa diffusion limitée. Cette démarche nécessite des outils de dépolymérisation spécifiques et efficaces. La réponse est apportée par des enzymes qui vont permettre de produire spécifiquement des oligosaccharides du fucane ou oligo-fucanes.

Il a été décrit, dans la demande de brevet WO 00/17215 des laboratoires Goëmar, une souche bactérienne nommée SW5 productrice d'endofucanases, ladite souche provenant de boues industrielles issues d'effluents riches en fucane sulfaté produits par une usine d'extraction d'alginates. Ladite souche appartient à la famille Flavobacteriaceae et produit une activité fucanolytique extracellulaire. L'activité endofucanase permet d'hydrolyser aléatoirement les liaisons glycosidiques du fucane de *Pelvetia canaliculata* et de produire des oligosaccharides. Aucune indication précise n'est donnée sur la distribution en taille des produits obtenus, cependant les chromatogrammes qui sont montrés indiquent une population de produits hétérogènes. Dans un article des mêmes auteurs (V. Descamps et al., « Isolation and culture of a marine bacterium degrading the sulfated from marine brown algae », Marine Biotechnology vol 8, pp 27-39, 2006), une faible activité fucanolytique est donnée pour cette souche. Il n'est pas possible selon les auteurs d'évaluer la production d'oligosaccharides par le test des sucres réducteurs bien connu de l'homme du métier. À la place, une analyse par électrophorèse sur gel est réalisée (C-PAGE), celle-ci permet de mettre en évidence l'apparition d'oligosaccharides de manière qualitative. Enfin, un seul type de fucane a été pris en compte dans cette étude, celui extrait de l'algue *Pelvetia canaliculata* appartenant à la famille des fucales. Or il existe d'autres familles d'algues brunes, notamment celle des laminariales dont les fucanes présentent des caractéristiques structurales différentes de celles des fucanes de fucale (Fucoidans - sulfated polysaccharides of brown algae 2009 Russ. Chem. Rev. 78 785-799). Une des différences majeures est la configuration des liaisons osidiques présentant une alternance de liaisons α(1-3) et α(1-4) dans les fucanes de fucales, et la répétition d'un seul type de liaison osidique α(1-3) dans les fucanes de laminaires. Cette différence est importante à prendre en compte car ces liaisons osidiques sont les sites d'action des activités enzymatiques de dépolymérisation de type fucanolytique.

Ici, les inventeurs ont pu mettre en évidence la dégradation du fucane appartenant aux deux familles fucale et laminariale, et en particulier le fucane *d'Ascophyllum nodosum* et le fucane de *Laminaria digitata,* par un mélange bactérien issu de la flore naturelle de l'algue brune *Ascophyllum nodosum.* Cette dégradation du fucane a été mise en évidence au cours de la culture de ce mélange bactérien et indique un niveau d'activité fucanolytique très élevé. Dans la présente invention, des consommations quantitatives (> 50 % en poids) de fucane présent à la concentration de 5 g/L sont démontrées par HPLC et par la mesure des sucres réducteurs. Le mélange bactérien décrit possède donc une action fucanolytique capable de s'exercer sur les différents types structuraux de fucanes.

Les inventeurs ont ainsi pu isoler, à partir de ce mélange bactérien, une souche bactérienne dont la caractérisation taxonomique indique qu'il s'agit d'un nouveau membre de la famille des Flavobacteriaceae. Ladite souche permet de dégrader également le fucane en produisant des oligofucanes avec un niveau d'activité élevée.

### RESUME DE L'INVENTION

La présente invention a donc pour objet de proposer une nouvelle voie de dépolymérisation microbiologique du fucane en utilisant une source enzymatique renouvelable et utilisable dans l'industrie.

À ce titre, la présente invention fournit une nouvelle souche bactérienne productrice d'enzymes à activité fucanolytique de la famille des Flavobacteriaceae.

L'invention propose également un mélange bactérien comprenant ladite souche, l'utilisation de ladite souche ou d'un mélange bactérien la comprenant pour la production d'enzymes à activité fucanolytique, le matériel enzymatique obtenu par la culture de ladite souche.

Elle a aussi pour objet un procédé de préparation d'oligofucanes, comprenant la mise en contact du fucane avec ladite souche, un mélange bactérien la comprenant ou le matériel enzymatique obtenu par la culture de ladite souche.

Elle décrit les fragments du fucane (ou oligofucanes) obtenus par ce procédé.

La présente invention divulgue aussi pour objet un procédé de préparation d'un matériel enzymatique doté d'activité fucanolytique extracellulaire par extraction du surnageant de culture de ladite souche bactérienne . Le matériel protéique ainsi obtenu peut être mis en oeuvre pour la production *in vitro* d'oligofucanes (sulfatés), indépendamment de toute culture bactérienne.

### DESCRIPTION DES FIGURES

Figure 1 : Croissance du mélange bactérien cultivé en fermenteur (DO 600 nm) et activité fucanolytique mesurée par HPLC-ES au cours de la culture avec du fucane (A) de fucale (*Ascophyllum nodosum)* et (B) de laminariale *(Laminaria digitata)*
Figure 2 : Croissance de la souche B1 cultivée en milieu Zobell (DO 600 nm) et activité fucanolytique mesurée par HPLC-ES (nRIU) au cours de la culture
Figure 3 : Suivi de la dépolymérisation du fucane *in vitro* catalysée par un extrait enzymatique issu du surnageant de culture du mélange bactérien selon l'invention
Figure 4 : Production d'extrémités réductrices au cours de la dépolymérisation du fucane *in vitro* catalysée par un extrait enzymatique issu du surnageant de culture du mélange bactérien selon l'invention
Figure 5 : Suivi par HPLC-ES de la dépolymérisation du fucane *in vitro* catalysée par un extrait enzymatique issu du surnageant de culture de la souche B1 selon l'invention (Témoin sans enzyme, Essai avec enzyme)
Figure 6 : Spectre de masse de la fraction oligosaccharidique issu du milieu de culture du mélange bactérien et purifiée par ultrafiltration selon l'invention

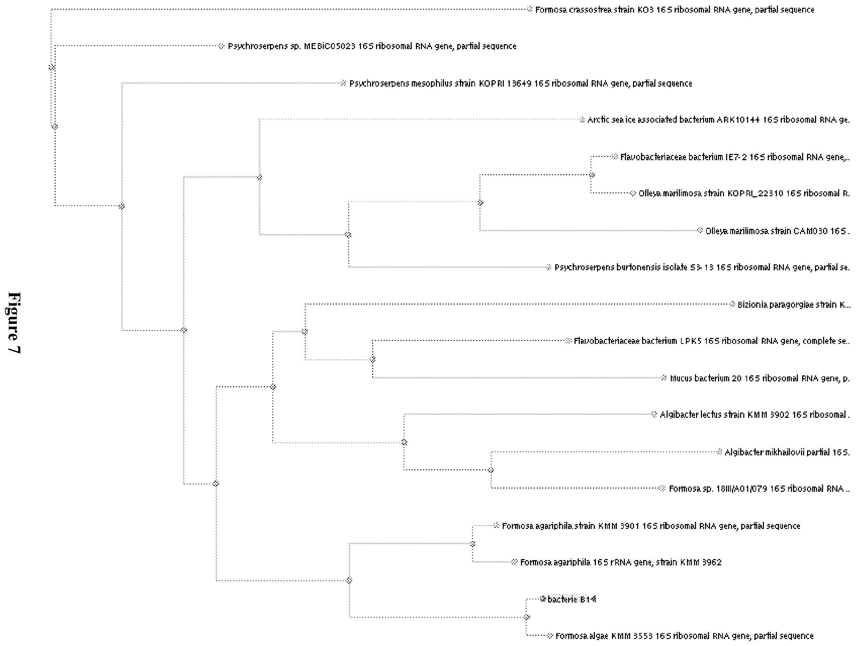

Figure 7 : Position phénétique de la souche B1

### DESCRIPTION DETAILLEE DE L'INVENTION

L'objet de la présente invention est tel que défini dans les revendications 1 à 5.

La présente invention concerne donc une nouvelle souche bactérienne productrice d'enzymes à activité fucanolytique de la famille des Flavobacteriaceae.

Cette souche est en particulier issue de la flore naturelle de l'algue brune *Ascophyllum nodosum.* Elle est plus particulièrement isolée d'un mélange bactérien collecté à partir de la flore naturelle de l'algue brune *Ascophyllum nodosum.* Plus particulièrement, afin de préparer un mélange bactérien contenant ladite souche bactérienne, une culture est réalisée en additionnant des morceaux coupés (finement) de l'algue brune *Ascophyllum nodosum* à un milieu sélectif. Le milieu sélectif comprend plus particulièrement un extrait de vase, des sels minéraux, de préférence NaCl (30g/l), K2HPO4 (2g/l), MgSO4 7H2O (1 g/l), NaNO3 (1g/l), FeCl2 (0,1 g/l), et du fucane, ce dernier étant de préférence issu *d'Ascophyllum nodosum* ou de *Laminaria digitata* (plus spécifiquement à une concentration de 5 g/L). Ce milieu pauvre comprenant du fucane permet d'exercer une pression de sélection et de sélectionner les micro-organismes capables de métaboliser le fucane.

L'isolement de la souche à partir du mélange bactérien peut être réalisé par diverses méthodes. Ainsi, il est possible d'étaler ledit mélange sur milieu solide selon la méthode des stries. Le milieu sélectif solide de même composition que le milieu sélectif liquide contient en outre 3,6% d'Agar pour permettre la gélification. On incube le mélange ainsi ensemencé pendant plusieurs jours, voire plusieurs semaines, et plus spécifiquement pendant trois semaines, de préférence à température ambiante (environ 22°C). Les colonies qui apparaissent sont très nombreuses et confluentes au départ des stries, puis se distancient de plus en plus les unes aux autres le long de la strie, pour apparaître individualisées à l'extrémité de la strie.

Les colonies bactériennes bien individualisées sont repérées et distinguées selon leur aspect morphologique. Chacune d'elles est ensuite repiquée et cultivée en milieu sélectif liquide pendant plusieurs jours, plus généralement pendant une semaine, de préférence à température ambiante (environ 22°C). À l'issue de cette culture, les souches peuvent être à nouveau repiquées selon la méthode des stries décrite ci-dessus afin de vérifier la présence d'un seul type morphologique. Le cas échéant, ce procédé d'isolement est réitéré à partir de ces milieux solides. Les souches isolées fucanolytiques et plus particulièrement la souche isolée B1 sont ensuite sélectionnées sur la base de la mesure de l'activité fucanolytique déterminée par la mesure HPLC-ES de la disparition du fucane présent en milieu de culture.

Afin d'obtenir une biomasse importante, la souche B1 ainsi isolée et sélectionnée est par la suite avantageusement cultivée en milieu riche référencé sous le nom de milieu Zobell connu de l'homme de métier. Le milieu de type Zobell additionné de fucane, de préférence issu d'*Ascophyllum nodosum* et/ou de *Laminaria digitata* (à une concentration de 5 g/L), comprend avantageusement de l'eau de mer filtrée (filtre 0,22 µm, stérilisation), un extrait de levure, de la peptone pepsique de viande et de l'eau osmosée. La composition du milieu Zobell est plus spécifiquement comme suit:

| | | |
|---|---|---|
| peptone pepsique de viande | | 5,0 g |
| Extrait de levure | | 1,0 g |
| Eau de mer filtrée | | 900 ml |
| Eau osmosée | q.s.p. | 11 |

La souche bactérienne B1 ainsi sélectionnée et isolée a été déposée le 03/09/2008 sous le numéro I-4070 dans la collection de l'Institut Pasteur dont l'adresse est la suivante : 25, rue du Docteur Roux, F-75724 Paris CEDEX 154

Les caractéristiques de la bactérie ainsi obtenue sont les suivantes : elle forme des colonies rondes de couleur jaune aux contours lisses. La bactérie selon l'invention est un bacille de type Gram négatif dont la taille est comprise entre 1,0 et 1,4 µm. D'autres caractéristiques physiologiques et biologiques de la souche isolée sont présentées dans les tableaux 1 et 2 ci-dessous.

**Tableau 1**

| Caractéristiques physiologiques de la bactérie isolée | |
|---|---|
| Caractères positifs | Caractères négatifs |
| β glucosidase | Réduction nitrates - nitrites |
| protéase | Réduction nitrates-azote |
| cytochrome oxydase | Formation indole |
| phosphatase alcaline | arginine dihydrolase |
| phosphatase acide | Uréase |
| estérase (C4) | β galactosidase |
| estérase lipase (C8) | lipase (C14) |
| leucine arylamidase | Cystine arylamidase |
| valine arylamidase | α chimotrypsine |
| trypsine | α galactosidase |
| Naphtol-AB-BI-phosphohydrolase | β galactosidase |
| α glucosidase | β glucuronidase |
| β glucosidase | α mannosidase |
| N-acetyl -β- glucosaminidase | |
| α fucosidase | |

**Tableau 2**

| Caractéristiques biologiques de la souche isolée | | |
|---|---|---|
| Caractères positifs | Caractères négatifs | Caractères négatifs |
| glycérol : assimilation du substrat/oxydation | caprate | salicine |
| erythritol : fermentation | adipate | cellobiose |
| D-arabinose : oxydation/fermentation | malate | lactose |
| D-xylose : assimilation du substrat/oxydation/fermentation | citrate | mélibiose |
| D-glucose : assimilation du substrat/oxydation/fermentation | phényl-acétate | saccharose |
| D-fructose : assimilation du substrat/oxydation/fermentation | L-arabinose | tréhalose |
| D-mannose : assimilation du substrat/oxydation/fermentation | ribose | inuline |
| Mannitol : assimilation du substrat/oxydation/fermentation | L-xylose | mélézitose |
| N-acétyl glucosamine : oxydation/ fermentation | adonitol | D-raffinose |
| amygdaline : fermentation | β méthyl-xyloside | xylitol |
| esculine : oxydation/fermentation | galactose | D turanose |
| maltose : assimilation du substrat/oxydation/fermentation | L-sorbose | D lyxose |
| amidon : assimilation du substrat/fermentation | Rhamnose | D tagatose |
| glycogène : fermentation | Dulcitol | D-fucose |
| β gentobiose : fermentation | Inositol | D arabitol |
| L fucose | sorbitol | L arabitol |
| | α méthyl-D-mannoside | gluconate |
| | α méthyl-D-glucoside | 2 ceto-gluconate |
| | arbutine | 5 cétogluconate |

Le séquençage de l'ARN 16S définit cette souche comme une nouvelle espèce appartenant au groupe Cytophaga-Flavobacterium (famille des Flavobacteriaceae). Les séquences ADNs SEQ ID N° 1 et/ou SEQ ID N° 2 correspondent à des copies d'ADN caractéristiques de l'ARN16s.

Comme spécifié ci-avant, la souche bactérienne selon l'invention est issue d'un mélange bactérien collecté à partir de la flore des thalles d'*Ascophyllum nodosum.*

La présente invention a également pour objet le mélange bactérien comprenant la bactérie décrite ci-dessus et issu de l'algue brune *Ascophyllum nodosum.*

Plus particulièrement, afin de préparer ledit mélange bactérien, une culture est réalisée en additionnant des morceaux coupés de l'algue brune *Ascophyllum nodosum* dans un milieu sélectif. Le milieu sélectif comprend plus particulièrement un extrait de vase enrichi en sels minéraux, de préférence NaCl (30g/l), K2HPO4 (2g/l), MgSO4 7H2O (1 g/l), NaNO3 (1g/l), FeCl2 (0,1 g/l), et du fucane, ce dernier étant de préférence issu d*'Ascophyllum nodosum* ou de *Laminaria digitata* (plus spécifiquement à une concentration de 5 g/l). Ce milieu pauvre permet d'exercer une pression de sélection et de favoriser le développement des microorganismes capables de métaboliser le fucane.

La présente invention a aussi pour objet le matériel enzymatique obtenu à partir de la souche bactérienne selon l'invention.

En particulier, le matériel enzymatique est obtenu à partir du surnageant de culture de ladite souche. Plus spécifiquement, le milieu de culture, en particulier le milieu sélectif, est centrifugé, notamment pour retirer le matériel biologique des bactéries (souche bactérienne, ...). Le surnageant obtenu est saturé, en particulier par précipitation fractionnée, à l'aide de sulfate d'ammonium, en particulier à 80% de saturation. Le culot protéique est alors récupéré, de préférence par centrifugation, puis dissout dans un tampon (e.g., tampon A Tris-HCl (50mM), NaCl (50 mM) à pH 7,5), et conservé à 4°C.

La présente invention décrit l'utilisation de ladite souche ou dudit mélange bactérien pour la production de matériels enzymatiques à activité fucanolytique. Elle divulgue une méthode de préparation de matériel enzymatique à activité fucanolytique comprenant la culture de ladite souche, la récupération du surnageant de culture et l'extraction des protéines contenues dans le surnageant.

Elle a aussi pour objet un procédé de préparation d'oligofucanes, comprenant la mise en contact du fucane, ce dernier étant de préférence issu d*'Ascophyllum nodosum* et/ou de *Laminaria digitata,* avec la souche, le mélange bactérien ou le matériel enzymatique, tels que définis dans la présente invention. Dans un mode particulier, le procédé comprend la culture de ladite souche ou dudit mélange bactérien, la préparation du matériel enzymatique à activité fucanolytique et la mise en contact du fucane avec le matériel enzymatique à activité fucanolytique.

Le substrat, à savoir le fucane, provient de deux seules sources connues à ce jour. Le fucane est un polysaccharide sulfaté qui entre dans la constitution des parois cellulaires des thalles d'algues brunes (Phéophycées). Ils sont également présents chez certains animaux marins, comme l'oursin et le concombre de mer.

Le fucane, obtenu généralement par extraction acide à partir des parois cellulaires des thalles, est constitué d'une population hétérogène de molécules qui comprend des polymères de fucose sulfatés de masse molaire moyenne élevée (supérieur à environ 50 000 g/mol).

Au sens de la présente invention, le terme « fucane » couvre les préparations de fucane de poids moléculaire élevé, et le terme « oligofucanes » couvre les fragments de fucane de poids moléculaire plus faible obtenus à partir du fucane de poids moléculaire élevé.

Plus particulièrement, le polysaccharide de type fucane est choisi parmi le fucane de haut poids moléculaire (c'est-à-dire de poids supérieur à environ 50 000), et les oligofucanes sont du fucane de bas poids moléculaires (c'est-à-dire de poids inférieur ou égal à environ 50 000, de préférence inférieur ou égal à environ 10 000 g/mol, et de manière encore plus préférée inférieur ou égal à environ 5 000 g/mol). En particulier, les oligofucanes sont des fucanes de poids moléculaire compris entre environ 1 000 et 10 000 g/mol, et de préférence entre 1 000 et 5 000 g/mol.

La capacité de la souche, du mélange bactérien ou du matériel enzymatique selon l'invention à produire des oligofucanes a été mise en évidence par le suivi de la dépolymérisation du fucane d*'Ascophyllum nodosum* et/ou de *Laminaria digitata* et par l'isolement d'oligo-fucanes analysés par spectrométrie de masse.

Dans le cas de la souche et du mélange bactérien, cette mise en évidence a été réalisée au cours de la culture en milieu additionné en fucane. L'analyse de la quantité de fucane dans le milieu a été réalisée par chromatographie liquide haute performance d'exclusion stérique (HPLC-ES) ainsi que par dosage de la quantité d'oses totaux.

Dans le cas de la souche bactérienne selon l'invention, l'analyse HPLC-ES montre une diminution du pic de fucane issu *d'Ascophyllum nodosum* de 70% en poids en 25 heures de culture de ladite souche et 50% de la quantité d'oses a disparu après 45 heures de culture.

La recherche de composés osidiques dans le surnageant de culture a permis de mettre en évidence la présence d'oligosaccharides dans le milieu de culture après 30 heures d'incubation. L'analyse par spectrométrie de masse de ces oligosaccharides montre qu'ils se composent de 1 à 4 fucoses (de préférence 1 à 3 sulfates), avec éventuellement la présence de xylose. Ces données montrent que la bactérie isolée à partir du thalle de l'algue *Ascophyllum nodosum* possède les systèmes enzymatiques capables de dégrader le fucane *Ascophyllum nodosum* et de le métaboliser.

Les essais de dégradation réalisés sur le fucane d'*Ascophyllum nodosum* (poids moléculaire : 100 000 g/mol) en utilisant le matériel protéique obtenu à partir de la souche bactérienne montrent une disparition du fucane de haut poids moléculaire d'environ 50% en poids en 42h d'incubation (mesuré par HPLC-ES). Cette consommation est corrélée à l'apparition de pics chromatographiques correspondant à des composés de bas poids moléculaire (poids moléculaire ≤ 5000 Da) de nature oligosaccharidiques. On démontre ainsi dans le surnageant de culture la présence de protéines exerçant une activité fucanolytique, et produites par la bactérie isolée selon l'invention.

Les essais de dégradation réalisés sur le fucane d'*Ascophyllum nodosum* et/ou de *Laminaria digitata* (100 000 g/mol) en utilisant le matériel protéique obtenu à partir du mélange bactérien montrent une diminution du signal du fucane de haut poids moléculaire d'environ 50% en poids en 3h de culture (mesuré par HPLC-ES). Cette consommation est corrélée à l'apparition de nouveaux pics chromatographiques correspondant à des composés de bas poids moléculaire définis comme des oligosaccharides. L'analyse par spectrométrie de masse par ionisation électrospray (ionisation négative) des oligosaccharides purifiés indique la formation d'une large gamme d'oligofucanes de bas poids moléculaire durant la réaction enzymatique variant du monosaccharide sulfaté au trisaccharide disulfaté.

La présente invention décrit ainsi les fragments du fucane (ou oligofucanes) obtenus selon le procédé décrit ci-dessus.

Ces fragments présentent généralement des degrés de polymérisation de 1 à 10, de préférence de 1 à 5, plus spécifiquement de 2 à 4, unités de fucose.

Ces oligofucanes plus particulièrement comprennent ou consistent en une fraction de 1 à 10 kD, de préférence 1 à 5 kD.

Ces oligofucanes plus particulièrement comprennent ou consistent en des oligofucanes ayant 1 à 4 fucoses et de 1 à 3 sulfates avec éventuellement la présence de xylose.

Les exemples ci-après sont fournis pour illustrer l'invention et ne doivent en aucun cas être considérés comme une limite à la portée de l'invention.

### EXEMPLES

### EXEMPLE 1: Mélange bactérien et caractérisations

### Préparation du milieu sélectif

On a procédé à l'extraction de 2 kg de vase avec de 2 litres d'eau distillée à 30 g/l de NaCl pendant 1 heure à 40°C sous agitation. Après décantation, le surnageant (1,3 litres) a été filtré successivement sur des filtres de 50, 1 et 0,5 µm. On arrive à un volume final de 1,25 litres. Ce filtrat a été complété par des sels minéraux, 2,5g de K2HPO4, 1,25g de NaNO3, 1,25g de MgSO4 7H2O, 2,5 ml d'une solution de chlorure de fer II à 2% et 6,25g de fucane issu *d'Ascophyllum nodosum* et/ou de *Laminaria digitata.* Le pH de la solution a été ajusté à 7,5 par environ 3ml de NaOH 1N avant d'être autoclavé à 121°C pendant 20 minutes. Pour obtenir un milieu solide, de l'agar agar a été ajouté au milieu à une concentration de 18 g/l.

### Préparation du mélange bactérien

Quelques branches *d'Ascophyllum nodosum* prélevées de manière stérile sur l'estran proche du sillon de Talbert à Pleubian (Côtes d'Armor, France) ont été coupées finement sous une hotte à flux, placées dans 100 ml de milieu sélectif tel que préparé ci-dessus et incubées à 25°C sous agitation. Après 7 jours d'incubation, 10 ml du mélange ainsi obtenu ont été introduits dans 100 ml de milieu sélectif. Après 7 jours d'incubation de cette culture nommée Exp1 b1, un test BSA (Bovine Serum Albumin) a été réalisé; celui-ci s'est avéré positif (la solution reste limpide) indiquant une dégradation du fucane. 10 ml de Exp1 b1 ont été introduits dans 100 ml de milieu sélectif tel que préparé ci-dessus pour donner la culture Exp1 b2 qui a été suivi par HPLC-ES toutes les 24 heures. Le test BSA est devenu positif après 48 heures d'incubation. Des échantillons de Exp1 b2 ont été conservés au congélateur (à -80°C), ce qui forme le mélange bactérien.

### Conditions de culture et activité

Une préculture (10 ml) en milieu sélectif a été réalisée à partir d'un échantillon dudit mélange bactérien conservé à -80°C. Cette préculture a été utilisée comme inoculum pour une culture (100 ml) en milieu sélectif (contenant du fucane issu *d'Ascophyllum nodosum*) réalisée sous agitation orbitale à 25°C pendant 20 heures. Cette culture a servi alors à ensemencer un Erlen contenant 1,1 litre de milieu sélectif agité sur un agitateur orbital (80-100 rpm) à 25°C pendant 30 heures.

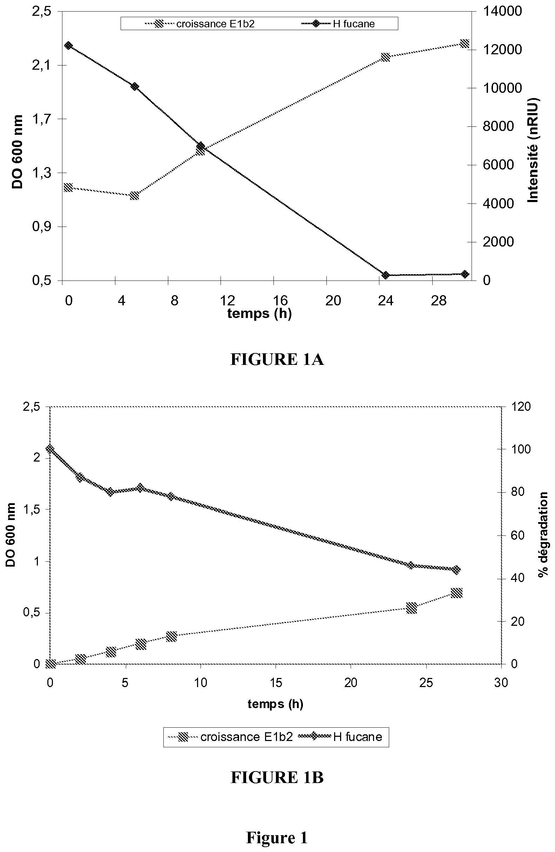

La croissance bactérienne a été mesurée par la densité optique (DO) du milieu à 600 nm. On observe ainsi que la DO du milieu passe de 1,1 à t=0 à environ 2,3 à t=30 heures (Fig. 1A).

L'activité fucanolytique (notée H fucane et exprimée en nRIU) mesurée par HPLC-ES montre une disparition du pic de fucane issu *d'Ascophyllum nodosum* après 24 heures d'incubation avec ledit mélange bactérien (Fig. 1A).

La même expérience a été réalisée dans les mêmes conditions avec un milieu sélectif contenant du fucane issu de *Laminaria digitata.*

La croissance bactérienne a été mesurée par la densité optique (DO) du milieu à 600 nm. On observe ainsi que la DO du milieu passe de 0 à t=0 à environ 0.7 à t=27 heures (Fig. 1B).

L'activité fucanolytique (notée H fucane et exprimée en % dégradation) mesurée par HPLC-ES montre une diminution de 46% du pic de fucane issu de *Laminaria digitata* après 24 heures d'incubation avec ledit mélange bactérien (Fig. 1B).

### EXEMPLE 2: Souche bactérienne isolée B1 et caractérisation

### Isolement de la souche bactérienne B1

Le mélange bactérien obtenu précédemment a été étalé sur milieu solide en boîte de Pétri selon la méthode des stries. Le milieu sélectif solide de même composition que le milieu sélectif liquide contient en outre 3,6% d'Agar. Les boîtes de Pétri ensemencées ont été incubées pendant trois semaines à température ambiante (environ 22°C).

Les colonies bactériennes bien individualisées ont été repérées et distinguées selon leur aspect morphologique. Chacune d'elles a ensuite été repiquée et cultivée en milieu sélectif liquide pendant une semaine, à température ambiante (environ 22°C). À l'issue de cette culture, les souches ont été à nouveau repiquées selon la méthode des stries décrite ci-dessus afin de vérifier la présence d'un seul type morphologique. Le cas échéant, ce procédé d'isolement a été réitéré à partir de ces boîtes de Pétri. Les bactéries isolées sélectionnées ont été celles démontrant une activité fucanolytique déterminée par la mesure HPLC-ES de la disparition du fucane présent en milieu de culture, et plus particulièrement la souche B1.

### Culture de la souche B1 en milieu Zobell

La culture de la souche B1 a été réalisée dans 100 ml de milieu Zobell fucane (noté ZF) contenant 5 g/l de fucane *d'Ascophyllum nodosum,* 5 g/l de peptone pepsique et 1 g/l d'extrait de levure dissout dans un mélange d'eau de mer filtrée et d'eau osmosée (dans un rapport volumique d'environ 9/1) à une température de 24°C.

Afin d'ensemencer cette culture, une préculture a été mise en oeuvre dans 20 ml de milieu ZF à partir d'une colonie prélevée sur boîte de Pétri. Après 24 h d'incubation, la préculture de B1 a une croissance normale et présente une densité optique (DO) proche de 4. La préculture a été étalée sur boîte de Pétri et ne présente aucune contamination.

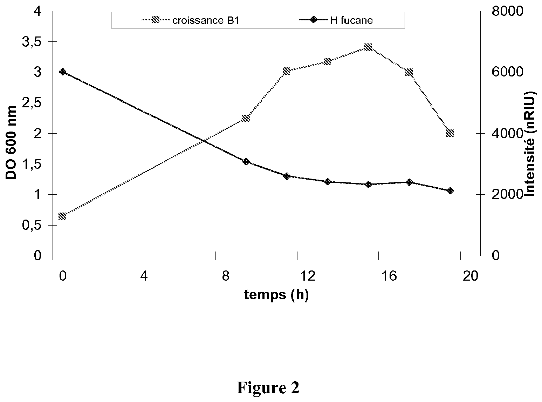

10 ml de préculture ont été utilisés pour ensemencer 100 ml de milieu de culture ZF. Les premiers prélèvements ont été effectués après 9 heures d'incubation afin d'éviter la phase de latence. Pour chaque culture, la DO à 600 nm et la hauteur du pic de fucane (noté H fucane et exprimée en nRIU) par HPLC-ES ont été mesurées et comparées à celles d'une culture témoin sans inoculum. Les résultats sont donnés à la figure 2.

La croissance bactérienne de la souche B1 atteint une valeur maximale de DO (600 nm) égale à 3,4. Le pic de fucane observe une diminution de 65% en 20 heures de culture (Fig. 2).

### EXEMPLE 3 : Matériel enzymatique issu du milieu de culture du mélange bactérien et propriétés

### Précipitation des protéines du surnageant de culture

La culture du mélange bactérien en milieu sélectif a été centrifugée à 15000 g pendant 15 min à 4°C pour enlever le culot bactérien. Le surnageant a été centrifugé une seconde fois dans les mêmes conditions pour enlever toute trace de résidus solides.
Les 960 ml de surnageant ont été saturés à 80% par du sulfate d'ammonium, l'ensemble a été agité doucement une nuit à 4°C. La solution saturée a été centrifugée à 15000 g pendant 10 min à 4°C pour récupérer un culot protéique. Celui-ci a été dissous dans 10 ml de tampon A (Tris HCl 50 mM, NaCl 50 mM pH 7,5) pour constituer la solution enzymatique qui a été conservée à 4°C.

### Essai enzymatique

120 mg de fucane *d'Ascophyllum nodosum* ont été dissous dans 27 ml de tampon A. La solution polysaccharidique a été additionnée de 3 ml de solution enzymatique. Le milieu réactionnel a été agité dans un bain marie à 30°C. L'analyse de la réaction a été réalisée par HPLC-ES et une mesure des oses réducteurs a été réalisée selon la méthode de Kidby & Davidson (Kidby D. K. and Davidson D.J., 1973, A convenient ferricyanide estimation of reducing sugars in the nanomole range. Anal. Biochem., 55, 321-325).

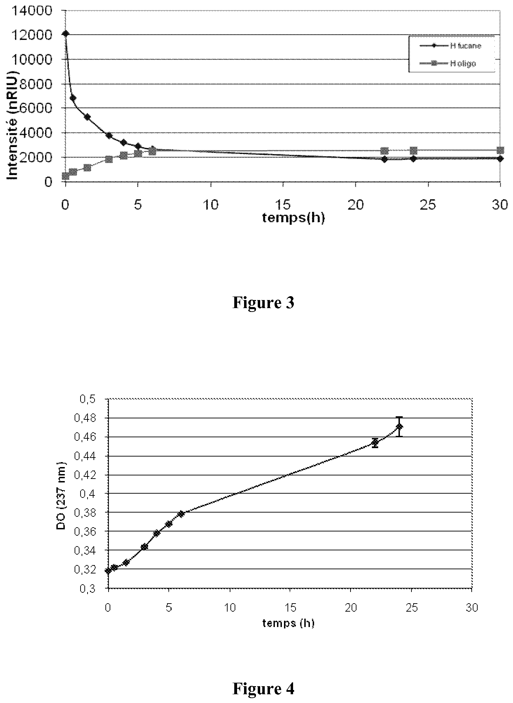

L'analyse par HPLC-ES (Figure 3) montre une diminution très rapide de la hauteur du pic de fucane dans les premières heures de réaction, indiquant une dégradation importante du polysaccharide. La hauteur minimale est atteinte après 24 heures de réaction. On observe ainsi une disparition du pic de fucane de 85%. En parallèle, on remarque l'augmentation d'un signal correspondant aux produits de réaction issus de la dégradation du polysaccharide dont les masses moléculaires se situent entre 3000 et 5000 Da (en équivalent de standards non chargés pullulanes). La hauteur de ce signal est multipliée par 5 après 24 heures de réaction.

L'analyse des oses réducteurs montre une augmentation de la quantité des extrémités réductrices dans le milieu réactionnel en fonction du temps (Figure 4). Cette observation montre la présence d'activité de type glycosidase capable de cliver le polysaccharide et de produire de nouvelles extrémités réductrices.

### EXEMPLE 4 : Matériel enzymatique issus du milieu de culture de la souche B1 et propriétés

### Précipitation des protéines du surnageant de culture

La culture de la souche B1 en milieu sélectif (V=120 ml) a été centrifugée pendant 15 minutes à 18335 g et à 4°C pour enlever le culot bactérien. Le surnageant (120 ml) a été filtré à 1,2 µm pour enlever toutes traces de résidus solides. Il a été saturé à 80% par du sulfate d'ammonium et l'ensemble a été agité doucement à 4°C pendant une nuit. La solution saturée a été centrifugée à 12000 tours par minute pendant 15 min à 4°C pour récupérer un précipité protéique. Celui-ci a été dissous dans 3 ml de tampon A (Tris HCl 50 mM, NaCl 50 mM, pH 7,5) pour constituer la solution enzymatique qui a été conservée à 4°C.

### Essai enzymatique

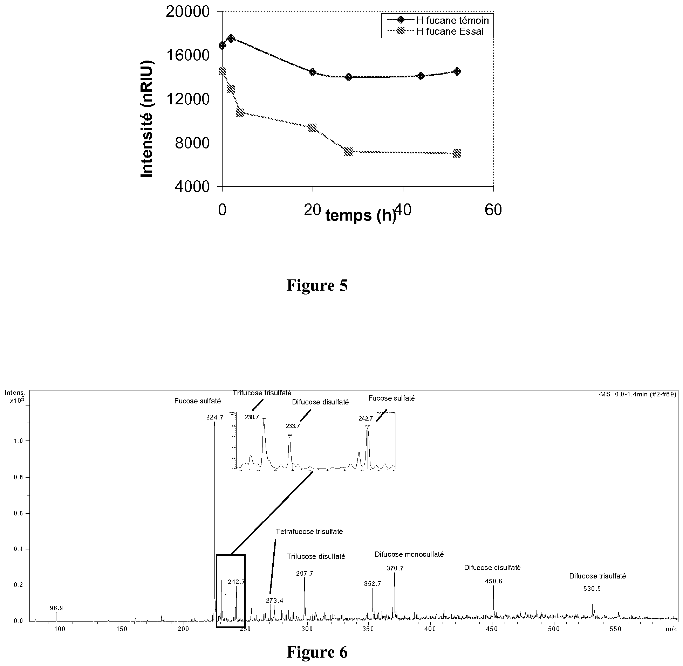

36 mg de fucane issu d'*Ascophyllum nodosum* ont été dissous dans 9 ml de tampon A. La solution polysaccharidique a été additionnée de 1,5 ml de solution enzymatique. Le mélange a été agité dans un bain marie à 27°C et analysé régulièrement par HPLC-ES. Les résultats sont comparés à un témoin dans lequel les enzymes ont été dénaturées préalablement à la chaleur (100°C ; 15 min). Les résultats sont présentés figure 5.

Dans le témoin, on observe une petite diminution du pic de fucane d'environ 15% lors des 20 premières heures de réaction. Celui-ci se stabilise ensuite autour d'une intensité de 14500 nRIU.

Dans l'essai, la hauteur du pic de fucane chute rapidement au cours des quatre premières heures d'incubation puis s'infléchit jusqu'à atteindre 51% en 30 heures de réaction.

Les enzymes du surnageant de culture de B1 en milieu Morinaga contiennent une activité fucanolytique qui permet de dégrader 50% du fucane en 30 heures d'incubation.

### EXEMPLE 5 : Analyse des oligosaccharides

### Purification des oligosaccharides

Les oligofucanes peuvent être isolés à partir des surnageants de culture dudit mélange ou de culture de la souche isolée B1 ou des milieux réactionnels enzymatiques. Pour cela, ils ont été filtrés, puis fractionnés par ultrafiltration : sur une membrane de seuil de coupure 10 000 (ou 10 kDa) afin d'enlever tous les composés de haute masse (> 15 kDa), puis sur une membrane 1 kDa afin d'enlever tous les petits composés (sel). La fraction notée 1-10 kDa restante contenant les oligosaccharides est purifiée par chromatographie d'exclusion stérique colonne superdex peptide (GE-Healthcare®) avec un éluant NH₄HCO₃ (0,1M, pH 8) à un débit 0,5 ml/min. Les composés d'intérêts élués (oligosaccharides sulfatés) sont détectés par réfractométrie et par conductimétrie. Les composés isolés sont lyophilisés et analysés par spectrométrie de masse.

### Analyse par spectrométrie de masse des oligosaccharides

Les oligosaccharides purifiés sont caractérisés par spectrométrie de masse en tandem par ionisation électrospray en mode négatif.
L'analyse de la fraction 1-10 kDa par spectrométrie de masse électrospray montre la présence d'oligosaccharides issus du fucane pouvant comporter de 2 à 5 fucoses, de 1 à 3 sulfates et la présence minoritaire d'oses neutres (Figure 6).

## Revendications

1. Souche bactérienne déposée le 3 septembre 2008 sous le numéro I-4070 dans la collection CNCM.

2. Mélange bactérien comprenant la souche bactérienne selon la revendication 1 et issu de la flore naturelle de l'algue brune *Ascophyllum nodosum.*

3. Matériel enzymatique à activité fucanolytique, obtenu à partir de la souche bactérienne selon la revendication 1, par extraction d'un surnageant de culture de la souche bactérienne selon la revendication 1.

4. Matériel enzymatique selon la revendication 3, **caractérisé en ce qu'**il est obtenu par l'extraction comprenant une étape de centrifugation et une étape de saturation du surnageant ainsi obtenu.

5. Procédé de préparation d'oligofucanes, comprenant la mise en contact du fucane, issu préférentiellement de Fucale (*Ascophyllum nodosum*) et/ou de Laminariale (*Laminaria digitata*)*,* avec la souche bactérienne selon la revendication 1, le mélange bactérien selon la revendication 2 ou le matériel enzymatique selon l'une des revendications 3 et 4.

## Patentansprüche

1. Bakterienstamm, hinterlegt am 3. September 2008 unter der Hinterlegungsnummer I-4070 bei der CNCM-Sammlung.

2. Mischung aus Bakterien, umfassend den Bakterienstamm gemäß Anspruch 1, die von der natürlichen Flora der Braunalge *Ascophyllum nodosum* stammt.

3. Enzymatisches Material mit Fucan abbauender Aktivität, erhalten aus dem Bakterienstamm gemäß Anspruch 1 mittels Extraktion eines Kulturüberstands des Bakterienstamms gemäß Anspruch 1.

4. Enzymatisches Material gemäß Anspruch 3, **dadurch gekennzeichnet, dass** es mittels Extraktion, umfassend einen Schritt der Zentrifugation und einen Schritt der Sättigung des folglich erhaltenen Überstands, erhalten wird.

5. Verfahren zur Präparation von Oligofucanen, umfassend Inkontaktbringen von Fucan, das vorzugsweise von Fucales (*Ascophyllum nodosum*) und/oder von Laminariales (*Laminaria digitata*) stammt, mit dem Bakterienstamm gemäß Anspruch 1, der Mischung aus Bakterien gemäß Anspruch 2 oder dem enzymatischen Material gemäß einem der Ansprüche 3 und 4.

## Claims

1. A bacterial strain deposited on the 3^{rd} of September 2008 under the number I-4070 in the CNCM collection.

2. A bacterial mixture comprising the bacterial strain according to claim 1 and originating from the natural flora of brown seaweed *Ascophyllum nodosum.*

3. An enzymatic material with a fucanolytic activity, obtained from the bacterial strain according to claim 1, by extracting a culture supernatant of the bacterial strain according to claim 1.

4. The enzymatic material according to claim 3, **characterised in that** it is obtained by the extraction comprising a step of centrifugation and a step of saturation of the supernatant thus obtained.

5. A process for preparing oligofucans, comprising contacting the fucan, preferably originating from Fucale (*Ascophyullum nodosum*) and/or Laminariale (*Laminaria digitata*)*,* with the bacterial strain according to claim 1, the bacterial mixture according to claim 2 or the enzymatic material according to one of claims 3 and 4.
